(19) Europäisches Patentamt
European Patent Office
Office européen des brevets

(11) Veröffentlichungsnummer : **0 495 749 A1**

(12) **EUROPÄISCHE PATENTANMELDUNG**

(21) Anmeldenummer : **92810003.1**

(22) Anmeldetag : **06.01.92**

(51) Int. Cl.$^5$ : **C07D 213/81,** A61K 31/44, C07D 213/78, C07D 213/83

(30) Priorität : **14.01.91 CH 85/91**

(43) Veröffentlichungstag der Anmeldung : **22.07.92 Patentblatt 92/30**

(84) Benannte Vertragsstaaten : **AT BE CH DE DK ES FR GB GR IT LI LU NL PT SE**

(71) Anmelder : **CIBA-GEIGY AG**
**Klybeckstrasse 141**
**CH-4002 Basel (CH)**

(72) Erfinder : **Stanek, Jaroslav, Dr.**
**Hangstrasse 9**
**CH-4144 Arlesheim (CH)**
Erfinder : **Caravatti, Giorgio, Dr.**
**Baselmattweg 145/C**
**CH-4123 Allschwil (CH)**
Erfinder : **Capraro, Hans-Georg, Dr.**
**Habsburgerstrasse 60**
**CH-4310 Rheinfelden (CH)**
Erfinder : **Frei, Jörg, Dr.**
**Buechring 36**
**CH-4434 Hölstein (CH)**

(54) **Bipyridyle.**

(57) Verbindungen der Formel I,

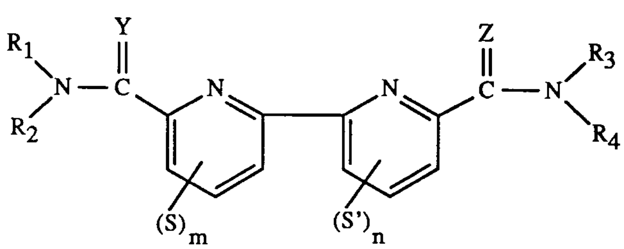

(I)

worin Y, Z, S, S′, m, n und $R_1$-$R_4$ die in der Beschreibung angegebenen Bedeutungen haben, weisen wertvolle pharmazeutische Eigenschaften auf und sind insbesondere gegen Protozoainfektionen wirksam. Sie werden in an sich bekannter Weise hergestellt.

EP 0 495 749 A1

Die Erfindung betrifft Verbindungen der Formel I

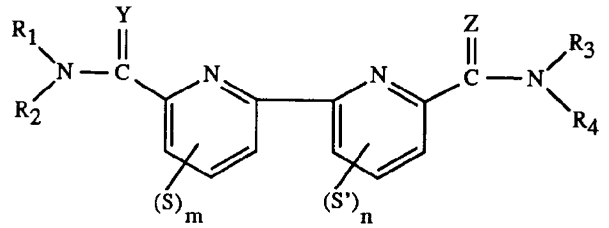

(I)

worin Y für $NR_5$, O oder S steht, Z für $NR_6$, O oder S steht, die Reste $R_2$, $R_4$, $R_6$, und $R_6$ unabhängig voneinander Wasserstoff oder Niederalkyl bedeuten und die Reste $R_1$ und $R_3$ unabhängig voneinander für Wasserstoff, Niederalkyl, Cycloalkyl, Arylniederalkyl, Aryl, freies oder funktionell abgewandeltes Carboxy, Hydroxy, verethertes oder verestertes Hydroxy oder unsubstituiertes oder mono- oder disubstituiertes Amino stehen; worin die Reste $R_1$ und $R_2$ zusammen auch Niederalkylen bedeuten können, worin die Reste $R_3$ und $R_4$ zusammen auch für Niederalkylen stehen können, worin die Reste $R_2$ und $R_5$ zusammen auch Niederalkylen bedeuten können, worin die Reste $R_4$ und $R_6$ zusammen auch für Niederalkylen stehen können; und worin S und S′ unabhängig voneinander einen von Wasserstoff verschiedenen Substituenten bedeuten; m für 0, 1, 2 oder 3 steht; n für 0, 1, 2 oder 3 steht, wobei jedoch die Summe aus m und n mindestens 1 ist; Tautomere davon, und Salze davon, Verfahren zur Herstellung dieser Verbindungen, pharmazeutische Präparate, die diese Verbindungen enthalten, die Verwendung dieser Verbindungen zur therapeurischen Behandlung des menschlichen oder tierischen Körpers oder zur Herstellung pharmazeutischer Präparate.

Tautomere können z.B. dann auftreten, wenn Y für $NR_6$ steht und $R_1$ und/oder $R_2$ Wasserstoff bedeuten: In diesem Fall kann der entsprechende Amidinrest, in Formel I als $-C(=Y)-NR_1R_2$ dargestellt, kann dann z.B. auch in den tautomeren Formen $-C(-YH)=NR_1$ oder $-C(-YH)=NR_2$ vorliegen. Ein weiteres Beispiel: Steht Z für $NR_6$ und ist $R_3$ und/oder $R_4$ Wasserstoff, so kann die entsprechende Amidinstruktur, in Formel I als $-C(=Z)-NR_3R_4$ dargestellt, ebenso in den tautomeren Formen $-C(-ZH)=NR_3$ oder $-C(-ZH)=NR_4$ vorliegen. Dem Fachmann ist das Auftreten solcher und ähnlicher Tautomere geläufig. Alle diese Tautomere werden von der allgemeinen Formel I mit umfasst.

Die vor- und nachstehend verwendeten Allgemeinbegriffe haben im Rahmen der vorliegenden Anmeldung vorzugsweise die folgenden Bedeutungen:

Das Präfix "Nieder" bezeichnet einen Rest bis und mit 7 und insbesondere bis und mit 4 Kohlenstoffatomen Alkyl ist z.B. $C_1$-$C_{20}$-Alkyl, und insbesondere Niederalkyl.

Niederalkyl ist z.B. n-Propyl, Isopropyl, n-Butyl, Isobutyl, sek-Butyl, tert-Butyl, n-Pentyl, Neopentyl, n-Hexyl oder n-Heptyl, vorzugsweise Ethyl und vor allem Methyl.

Cycloalkyl enthält z.B. 3 bis 8, vorzugsweise 5 oder 6, Ringkohlenstoffatome und ist z.B Cyclopropyl, Cyclopentyl, Cyclohexyl oder Cycloheptyl.

Arylniederalkyl ist vorzugsweise Phenylniederalkyl und insbesondere Benzyl.

Aryl ist z.B. Phenyl oder Naphthyl, wie 1- oder 2-Naphthyl. Die Phenyl- oder Naphthylreste können unsubstituiert oder substituiert sein. Aryl ist bevorzugt Phenyl, das unsubstituiert oder durch Niederalkyl, Hydroxy, Niederalkoxy, Halogen und/oder Trifluormethyl substituiert ist, und in erster Linie Phenyl.

Freies oder funktionell abgewandeltes Carboxy ist bevorzugt Cyano, ferner auch z.B. Carboxy, verestertes Carboxy, wie z.B. Niederalkoxycarbonyl, oder amidiertes Carboxy, wie z.B. Carbamoyl ($-CONH_2$), N-Niederalkylcarbamoyl oder N,N-Diniederalkylcarbamoyl.

Verethertes Hydroxy ist z.B. Alkoxy, wie $C_1$-$C_{20}$-Alkoxy, und insbesondere Niederalkoxy. Verestertes Hydroxy ist z.B. Niederalkanoyloxy. Monosubstituiertes Amino ist z.B. Niederalkylamino. Disubstituiertes Amino ist z.B. Diniederalkylamino, $C_4$-$C_6$-Alkylenamino, z.B. Piperidino, Oxa-$C_3$-$C_5$-alkylenamino, z.B. Morpholino, Thia-$C_3$-$C_5$-alkylenamino, z.B. Thiomorpholino, oder Aza-$C_3$-$C_5$-alkylenamino, welches unsubstituiert oder am Azastickstoff Niederalkyl-substituiert ist, z.B. Piperazino oder 4-Niederalkylpiperazino. Die Begriffe "Oxa-$C_3$-$C_5$", "Thia-$C_3$-$C_5$" und "Aza-$C_3$-$C_5$" oben sind so definiert, dass das entsprechende Heteroatom zusätzlich zu den 3-5 Kohlenstoffatomen (und nicht: anstelle eines der Kohlenstoffatome) vorliegt. Bevorzugt bedeutet disubstituiertes Amino Diniederalkylamino.

Niederalkylen gebildet aus den Gruppen $R_1$ und $R_2$ bzw. $R_3$ und $R_4$ ist bevorzugt $C_2$-$C_7$-Alkylen und insbesondere $C_4$-$C_5$-Alkylen, z.B. 1,4-Butylen oder 1,5-Pentylen.

Niederalkylen gebildet aus den Gruppen $R_2$ und $R_5$ bzw. $R_4$ und $R_6$ ist bevorzugt $C_2$-$C_5$-Alkylen und insbesondere $C_2$-$C_3$-Alkylen, z.B. 1,2-Ethylen oder 1,3-Propylen.

Niederalkylendioxy ist z.B. Methylendioxy oder Ethylendioxy ($-O-CH_2-CH_2-O-$).

Halogen bedeutet z.B. Fluor oder Iod, insbesondere Brom und vor allem Chlor.

Niederalkanoyl ist z.B. Acetyl, Propionyl oder Pivaloyl, ferner auch z.B. Formyl.

Beispiele für einen von Wasserstoff verschiedenen Substituenten S bzw. S′sind: gegebenenfalls substituierte Kohlenwasserstoffradikale, wie entsprechende aliphatische, cycloaliphatische, cycloaliphatisch-aliphatische, aromatische oder araliphatische Kohlenwasserstoffradikale, wie Alkyl, insbesondere Niederalkyl, Niederalkenyl, Niederalkinyl, Niederalkylen, das als bivalenter Substituent in zwei benachbarten Positionen an den Ring gebunden ist, Cycloalkyl, Phenylniederalkyl, Cycloalkylniederalkyl oder Phenyl (Substituenten solcher Kohlenwasserstoffradikale sind z.B. Hydroxy, verethertes oder verestertes Hydroxy, wie Niederalkoxy, Halogen-niederalkoxy, Niederalkenyloxy, Niederalkinyloxy, Niederalkylendioxy oder Niederalkanoyloxy; Halogen, Niederalkyl, Trifluormethyl, Carboxy und/oder funktionell modifiziertes Carboxy, wie verestertes Carboxy, z.B. Niederalkoxycarbonyl, amidiertes Carboxy, wie Carbamoyl, Niederalkylcarbamoyl oder Diniederalkylcarbamoyl, oder Cyano); Trifluormethyl; Hydroxy; verethertes oder verestertes Hydroxy, wie Alkoxy, insbesondere Niederalkoxy, Halogen-niederalkoxy, Niederalkenyloxy, Halogen-niederalkenyloxy, Niederalkinyloxy, Phenyloxy oder Phenylniederalkoxy (wobei die beiden letztgenannten Substituenten im Phenylring unsubstituiert oder z.B. durch Niederalkyl, Hydroxy, Niederalkoxy, Halogen und/oder Trifluormethyl substituiert sind), Niederalkylendioxy, das als bivalenter Substituent in zwei benachbarten Positionen an den Ring gebunden ist, oder Niederalkanoyloxy; Halogen, Nitro; Amino, substituiertes Amino, wie Niederalkylamino, Diniederalkylamino, N-Niederalkyl-N-phenylniederalkylamino, Niederalkylenamino, Oxa-, Thia- oder Aza-niederalkylenamino (wobei das Aza-Stickstoffatom unsubstituiert oder substituiert ist, vorzugsweise durch Niederalkyl, aber auch z.B. durch Phenyl, Phenylniederalkyl oder durch Acyl, z.B. Niederalkanoyl oder Benzoyl) oder Acylamino, z.B. Niederalkanoylamino; Formyl; Acyl, wie Niederalkanoyl; Carboxy; funktionell modifiziertes Carboxy, wie verestertes Carboxy, z.B. Niederalkoxycarbonyl, oder amidiertes Carboxy, wie Carbamoyl, Niederalkylcarbamoyl oder Diniederalkylcarbamoyl, oder Cyano; Sulfo ($-SO_3H$); funktionell modifiziertes Sulfo, wie Sulfamoyl, Niederalkylsulfamoyl, Diniederalkylsulfamoyl oder Phenylsulfamoyl; und verethertes Mercapto, das gegebenenfalls oxidiert sein kann, wie Niederalkylthio, Niederalkylsulfinyl oder Niederalkylsulfonyl. Bevorzugte Substituenten S bzw. S′ sind Niederalkyl, Niederalkoxy und Halogen.

Steht das Symbol m (bzw. n) in einer Verbindung der Formel I für 2 oder 3, so können die entsprechenden 2 oder 3 Substituenten S (bzw. S′) gleich oder verschieden sein.

Salze von erfindungsgemässen Verbindungen sind in erster Linie pharmazeutisch verwendbare, nicht-toxische Salze. Beispielsweise können Verbindungen der Formel I mit basischen Gruppen Säureadditionssalze, z.B. mit anorganischen Säuren, wie Salzsäure, Schwefelsäure oder Phosphorsäure, oder mit geeigneten organischen Carbon- oder Sulfonsäuren, z.B. Essigsäure, Fumarsäure oder Methansulfonsäure, oder z.B. mit Aminosäuren, wie Asparaginsäure oder Glutaminsäure, bilden. Bei Anwesenheit von mehr als einer basischen Gruppe können Mono- oder Polysalze gebildet werden. Verbindungen der Formel I mit einer sauren Gruppe, z.B. Carboxy, und einer basischen Gruppe, z.B. Amino, können z.B. in Form von inneren Salzen, d.h. in zwitterionischer Form vorliegen, oder es kann ein Teil des Moleküls als inneres Salz, und ein anderer Teil als normales Salz vorliegen.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch verwendbaren, nicht- toxischen Salze, die deshalb bevorzugt sind.

Je nach den strukturellen Gegebenheiten können die Verbindungen der vorliegenden Erfindung in Form von Isomerengemischen oder von reinen Isomeren vorliegen.

Die erfindungsgemässen Verbindungen weisen wertvolle, insbesondere pharmakologisch verwendbare, Eigenschaften auf. Insbesondere haben sie eine starke, spezifische Hemmwirkung auf das Enzym S-Adenosylmethionindecarboxylase (SAMDC). SAMDC spielt als ein Schlüsselenzym eine wichtige Rolle bei der Polyaminbiosynthese, die in praktisch allen Zellen von Säugetieren, einschliesslich Menschen, abläuft. Durch SAMDC wird die Polyaminkonzentration in der Zelle reguliert. Eine Hemmung des Enzyms SAMDC hat eine Verringerung der Polyaminkonzentration zur Folge. Da eine Verringerung der Polyaminkonzentration eine Hemmung des Zellwachstums bewirkt, ist es möglich, durch Verabreichung von SAMDC-hemmenden Substanzen das Wachstum sowohl von eukaryotischen als auch prokaryotischen Zellen zu hemmen und sogar Zellen abzutöten oder das Einsetzen der Zelldifferenzierung zu hemmen.

Die Hemmung des Enzyms SAMDC kann z.B. mit der Methode von H.G. Williams-Ashmann und A. Schenone, Biochem.Biophys.Res.Communs. 46, 288 (1972) nachgewiesen werden. Die Verbindungen der Erfindung weisen $IC_{50}$-Werte von minimal etwa 1 $\mu$M auf.

Ein Vorteil der erfindungsgemässen Verbindungen besteht darin, dass sie nur in geringem Masse im Vergleich zu ihrer starken Hemmwirkung auf SAMDC die Diaminoxidase inhibieren und gut verträglich sind. Die Hemmung der Diaminoxidase ist nach J. Jaenne und D.R. Morris, Biochem. J. 218, 974 (1984) ungünstig, da sie zur Akkmulation von Putrescin und einer indirekten SAMDC-Aktivierung führen kann.

Daher sind die Verbindungen der Formel I z.B. nützlich zur Behandlung benigner und maligner Tumoren. Sie können Tumorregressionen bewirken und ferner die Verbreitung von Tumorzellen sowie das Wachstum von Mikometastasen verhindern. Des weiteren können sie z.B. zur Behandlung von Protozoainfektionen, wie etwa Trypanosomiasis, Malaria oder durch Pneumocystis carinii verursachte Lungenentzündung, dienen.

Beispielsweise kann die Wirksamkeit der Verbindungen der Formel I und ihrer Salze, bei den nachfolgenden Testbeschreibungen als Testsubstanzen bezeichnet, gegen Trypanosomiasis in vitro anhand des "Long Incubation - Low-Inoculation"-Tests (LLIT) und des $^3$H-Hypoxanthin-Einbautestes nachgewiesen werden.

Der LLIT dient zur Ermittlung einer minimalen Hemmkonzentration und einer maximal tolerierten Konzentration der Testsubstanzen bei Einwirkung auf Trypanosomen (Blutstromformen von Trypanosoma brucei rhodesiense, einem humanpathogenen Erreger der afrikanischen Schlafkrankheit) während längerer Zeit (Langzeitinkubation). Hierzu werden in einer Mikrotiterplatte Trypanosomen mit verschiedenen Konzentrationen der jeweiligen Testsubstanz inkubiert. Die Inkubationsbedingungen sind wie folgt definiert: Blutstromformen von Trypanosoma brucei rhodesiense werden bei 37 °C und 5 % $CO_2$ axenisch kultiviert in einem Medium folgender Zusammensetzung: 9,75 g MEM power ("Minimum Essential Medium"; Gibco, USA; siehe auch Eagle, H., Science 130, 432 (1959)), 6 g HEPES (N-2-Hydroxyethylpiperazin-N'-2-ethansulfonsäure, Calbiochem GmbH, Bundesrepublik Deutschland), 1 g Glucose, 2,2 g $NaHCO_3$, 10 ml MEM NEAA (nichtessentielle Aminosäurenlösung), gelöst in destilliertem Wasser ad 1000 ml und mit 4 N NaOH auf pH 7,4 eingestellt, 15 % hitzeinaktiviertes Pferdeserum (Inaktivierung 30 Minuten bei 56 °C), Baltz-Komponenten (0,1 mM Hypoxanthin, 0,016 mM Thymidin, 2 mM Natriumpyruvat und 0,2 mM Mercaptoethanol, und 10 µg Gentamycin pro ml Medium bei An- oder Abwesenheit von verschiedenen Konzentrationen der Testsubstanzen. Nach 4 Tagen Expositionszeit wird der Test visuell ausgewertet und die MIC und die MTC bestimmt. Unter MIC versteht man die "Minimal Inhibitory Concentration" (die minimale Hemmkonzentration), also die niedrigste Konzentration der Testsubstanzen, bei der keine Zellen mit einer normalen Morphologie oder Bewegungsfähigkeit mehr vorhanden sind. Unter MTC versteht man die "Maximal Tolerable Concentration" (maximal tolerierte Konzentration), also die höchste Konzentration der Testsubstanz, bei der im Vergleich zur Kontrolle ohne Testsubstanz noch mindestens 80% der Trypanosomen überleben. Eine typische MIC der Testsubstanzen der Formel I, ihrer Tautomeren oder ihrer Salze liegt nahe bei 0,5 µM oder mehr, eine typische MTC nahe bei 0,25 µM oder mehr.

Der $^3$H-Hypoxanthin-Einbautest dient zur Bestimmung der Sensitivität von Trypanosomen bei Kurzzeitinkubation einer Substanz. Das Prinzip des Einbautestes beruht darauf, dass die Trypanosomen mit der radioaktiv markierten Purinbase $^3$H-Hypoxanthin inkubiert werden. Die Trypanosomen können diese Base nicht selbst synthetisieren und sind deshalb auf eine externe Quelle angewiesen. Der Einbau der Purinbase ist proportional zum Wachstum und der metabolischen Aktivität der Trypanosomen. Wie beim LLIT werden die Trypanosomen in einer Mikrotiterplatte mit absteigendem Konzentrationen der jeweiligen Testsubstanz inkubiert. Die Vorinkubation dauert 24 Stunden, die Inkubation mit der Purinbase weitere 16 Stunden (Gesamtdauer 40 Stunden). Die Zellen werden geerntet, gewaschen und auf ein Filterpapier transferiert, mit Szintillationsflüssigkeit versetzt und in einem β-Counter gemessen. Die Kontrolle (Ansatz ohne Testsubstanz der Formel I) wird als 100 % Einbau der Purinbase beziehungsweise der metabolischen Aktivität festgelegt, die übrigen Ansätze mit Testsubstanz der Formel I werden in Prozent der Kontrolle angegeben. Eine $IC_{50}$ wird bestimmt als die Konzentration, bei der die Werte der Ansätze mit Testsubstanz gegenüber der Kontrolle einen um 50 % verminderten Einbau/metabolische Aktivität zeigen (Lit.: Brun, R., et al, Acta Tropica 46, 361 - 368 (1989).

Ein typischer $IC_{50}$-Wert der Verbindungen der Formel I, ihrer Tautomeren oder ihrer Salze liegt nahe bei 0,25 µM oder mehr.

Als selektive SAMDC-Hemmer können die Verbindungen der Formel I allein oder auch in Kombination mit anderen pharmakologisch wirksamen Substanzen angewendet werden. Zu denken ist z.B. an eine Kombination mit (a) Inhibitoren anderer Enzyme der Polyaminbiosynthese, z.B. Ornithindecarboxylasehemmern, (b) Inhibitoren der Proteinkinase C, (c) Inhibitoren der Tyrosinproteinkinase, (d) Cytokinen, (e) negativen Wachstumsregulatoren, (f) Aromatasehemmern, (g) Antiöstrogenen oder (h) klassischen zytostatischen Wirkstoffen.

Bevorzugt sind die Verbindungen der Formel I, worin Y für NH, O oder S steht, Z für NH, O oder S steht, die Reste $R_2$ und $R_4$ unabhängig voneinander Wasserstoff oder Niederalkyl bedeuten und die Reste $R_1$ und $R_3$ unabhängig voneinander für Wasserstoff, Niederalkyl, $C_3$-$C_8$-Cycloalkyl, Phenylniederalkyl, Phenyl, Carboxy, Hydroxy oder Amino stehen; worin die Reste $R_1$ und $R_2$ zusammen auch $C_2$-$C_7$-Alkylen bedeuten können, worin die Reste $R_3$ und $R_4$ zusammen auch für $C_2$-$C_7$-Alkylen stehen können; und worin S und S' unabhängig voneinander $C_1$-$C_{20}$-Alkyl, $C_3$-$C_8$-Cycloalkyl, Phenylniederalkyl, $C_3$-$C_8$-Cycloalkylniederalkyl, Phenyl, $C_1$-$C_{20}$-Alkoxy, Phenylniederalkoxy, Phenyloxy, Halogen oder Niederalkylthio bedeuten; m für 0 oder 1 steht; und n für 0 oder 1 steht;

wobei jedoch die Summe aus m und n mindestens 1 ist;

wobei in den obigen Definitionen Phenylgruppen unsubstituiert oder durch Niederalkyl, Hydroxy, Niederalkoxy,

Halogen und/oder Trifluormethyl substituiert sind; Tautomere davon, und Salze davon.

Besonders bevorzugt sind die Verbindungen der Formel I, worin Y für NH, O oder S steht, Z für NH, O oder S steht, die Reste $R_2$ und $R_4$ unabhängig voneinander Wasserstoff oder Niederalkyl bedeuten und die Reste $R_1$ und $R_3$ unabhängig voneinander für Wasserstoff, Niederalkyl, $C_3$-$C_8$-Cycloalkyl, Phenylniederalkyl, Phenyl, Carboxy, Hydroxy oder Amino stehen; worin die Reste $R_1$ und $R_2$ zusammen auch $C_2$-$C_7$-Alkylen bedeuten können, worin die Reste $R_3$ und $R_4$ zusammen auch für $C_2$-$C_7$-Alkylen stehen können; und worin S und S′ unabhängig voneinander Niederalkyl, Phenylniederalkyl, Niederalkoxy, Halogen oder Niederalkylthio bedeuten; m für 0 oder 1 steht; und n für 0 oder 1 steht; wobei jedoch die Summe aus m und n mindestens 1 ist; Tautomere davon, und Salze davon.

Ganz besonders bevorzugt sind die Verbindungen der Formel I, worin Y für NH, O oder S steht, Z für NH, O oder S steht, die Reste $R_2$ und $R_4$ Wasserstoff oder Niederalkyl bedeuten, die Reste $R_1$ und $R_3$ für Wasserstoff, Niederalkyl, $C_5$-$C_6$-Cycloalkyl oder Hydroxy stehen, S und S′ Niederalkyl, Niederalkoxy oder Halogen bedeuten, und m und n für 1 stehen; Tautomere davon, und pharmazeutisch verwendbare Salze davon.

In erster Linie bevorzugt sind die Verbindungen der Formel I, worin Y für NH steht, Z für NH steht, die Reste $R_2$ und $R_4$ Wasserstoff bedeuten, die Reste $R_1$ und $R_3$ für Wasserstoff, Niederalkyl, $C_5$-$C_6$-Cycloalkyl oder Hydroxy stehen, S und S′ in 4- bzw. 4′-Stellung angeknüpft sind und Niederalkyl, Niederalkoxy oder Halogen bedeuten, und m und n für 1 stehen, Tautomere davon, und pharmazeutisch verwendbare Salze davon.

Als Untergruppen aus einer Gruppe von Verbindungen der Formel I sind jeweils hervorzuheben: (a) Verbindungen der Formel I, worin die Gruppen

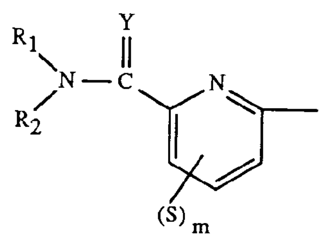

und

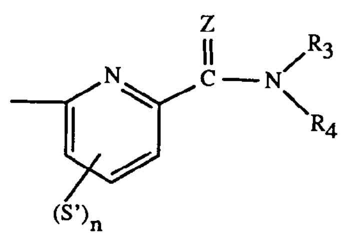

identisch sind; (b) Verbindungen der Formel I, worin $R_1$, $R_2$, $R_3$ und $R_4$ Wasserstoff bedeuten; (c) Verbindungen der Formel I, worin die Substituenten S und S′ in 4-Stellung (bzw. 4′-Stellung) angeknüpft sind, (d) Verbindungen der Formel I, worin Y und Z für NH stehen.

Die Erfindung betrifft vor allem die in den Beispielen beschriebenen spezifischen Verbindungen und pharmazeutisch anwendbare Salze davon.

Die Verbindungen der Formel I können in an sich bekannter Weise hergestellt werden, indem man z.B. in einer Verbindung der Formel II

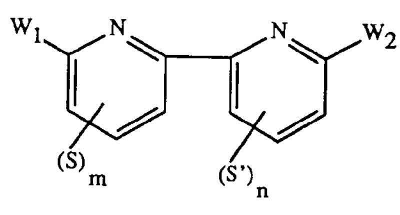

(II)

worin $W_1$ und $W_2$ jeweils einen in die Gruppe -C(=Y)$NR_1R_2$ bzw. -C(=Z)$NR_3R_4$ überführbaren Rest bedeuten, die Reste $W_1$ und $W_2$ in die Gruppe -C(=Y)$NR_1R_2$ bzw. -C(=Z)$NR_3R_4$ überführt; wobei die Symbole Y, Z, $R_1$-$R_4$, S, S′, m und n die unter Formel I angegebene Bedeutung haben; und gewünschtenfalls eine erhaltene Verbindung der Formel I in eine andere Verbindung der Formel I umwandelt, und/oder ein erhaltenes Salz in die freie Verbindung oder in ein anderes Salz umwandelt, und/oder eine erhaltene freie Verbindung der Formel I mit salzbildenden Eigenschaften in ein Salz umwandelt.

In der folgenden näheren Beschreibung des Verfahrens haben die Symbole Y, Z, $R_1$ -$R_6$, S, S′, m und n jeweils die unter Formel I angegebene Bedeutung, sofern nichts anderes angegeben ist.

In den Zwischenprodukten der Formel II bedeuten $W_1$ und $W_2$ z.B. freies oder funktionell abgewandeltes Carboxy, insbesondere Halogencarbonyl, Carbamoyl, N-Niederalkylcarbamoyl, Cyan, einen Imino-niederalkylester [-C(=NH)-OAlk (Alk ≙ Niederalkyl)] oder Imino-niederalkylthiolester [-C(=NH)-SAlk].

Die Gruppen $W_1$ und/oder $W_2$ in einer Verbindung der Formel II können bei der Herstellung von (Mono-

oder Di-)Amidinen der Formel I (Y ≙ $NR_5$ und/oder Z ≙ $NR_5$) z.B. bedeuten: einen Imino-niederalkylester (≙ Imino-niederalkylether) oder Imino-niederakylthiolester bzw. ein Säureadditionssalz davon, z.B. -C(=NH)-$OC_2H_5$ oder -C(=NH)-$SC_2H_5$ bzw. -C(=NH)-$OC_2H_5$·HCl oder -C(=NH)-$SC_2H_5$·HI; Cyano oder N-Niederalkylcarbamoyl.

Durch die Umsetzung von (Mono- oder Di-)Imino-niederalkylestem bzw. (Mono- oder Di-)Imino-niederalkylthiolestem der Formel II (als Säureadditionssalze) mit Ammoniak oder primären bzw. sekundären Aminen erhält man die unsubstituierten bzw. mono- oder disubstituierten (Mono- oder Di-)Amidine der Formel I.

Analog ist es möglich, die (Mono- oder Di-)Imino-niederalkylester bzw. (Mono- oder Di-)Imino-niederalkylthiolester der Formel II in freier Form, welche bevorzugt in situ hergestellt werden, mit einem Ammoniumsalz, z.B. $^{\oplus}NH_4Cl^{\ominus}$, oder einem Ammoniumsalz eines primären oder sekundären Amins umzusetzen, wobei man ebenfalls die unsubstituierten bzw. mono- oder disubstituierten (Mono- oder Di-)Amidine der Formel I erhält.

(Mono- oder Di-)Cyanoverbindungen der Formel II können z.B. durch Umsetzung mit einem Alkalimetallamid, z.B. $KNH_2$, oder durch Reaktion mit einem primären oder sekundären (Di-)niederalkylammoniumhalogenid, z.B. $^{\oplus}NH_3CH_3\ Cl^{\ominus}$, oder einem Salz des Hydroxylamins, z.B. $^{\oplus}NH_3OH\ Cl^{\ominus}$, in ein gegebenenfalls mono- oder disubstituiertes (Mono- oder Di-)Amidin der Formel I überführt werden.

(Mono- oder Di-)N-hydroxyamidine der Formel I lassen sich durch Reduktion, z.B. katalytische Hydrierung, etwa mit $H_2$/Raney-Nickel, in (Mono- oder Di-)Amidine der Formel I überführen [vgl. DE-OS-2 705 609 oder J. Med. Chem. 15, 182- 186 (1972)].

Verbindungen der Formel II, worin $W_1$ und/oder $W_2$ N-Niederalkylcarbamoyl bedeuten, lassen sich z.B. durch Umsetzung mit $POCl_3$ oder $PCl_5$ in die entsprechenden Imidsäurechloride [-C(=NH-Alk)-Cl] überführen, welche nach Reaktion mit Ammoniak beziehungsweise einem primären oder sekundären Amin substituierte (Mono- oder Di-)Amidine der Formel I ergeben [vgl. Chem. Abstr. 81, 91185a (1974)].

Verbindungen der Formel I, worin die Reste $R_2$ und $R_5$ zusammen und/oder die Reste $R_4$ und $R_5$ zusammen Niederalkylen bedeuten, lassen sich z.B. dadurch herstellen, dass man eine Verbindung der Formel II, worin $W_1$ und/oder $W_2$ Cyano bedeuten, mit einem $\alpha,\omega$-Diaminoniederalkan, z.B. 1,2-Diaminoethan, - bevorzugt in Gegenwart von katalytischen Mengen Schwefelkohlenstoff - umsetzt.

Die Gruppen $W_1$ und/oder $W_2$ in einer Verbindung der Formel II können bei der Herstellung von (Mono- oder Di-)Carbamoylverbindungen der Formel I (Y ≙ O und/oder Z ≙ O) z.B. bedeuten: Carboxy, Halocarbonyl (z.B. -COCl), Niederalkoxycarbonyl oder Cyano. Die Bildung von gegebenenfalls mono- oder disubstituierten (Mono- oder Di-)Carbamoylverbindungen der Formel I aus entsprechenden Zwischenprodukten der Formel II, worin $W_1$ und/oder $W_2$ Carboxy, Halocarbonyl oder Niederalkoxycarbonyl bedeuten, durch Umsetzung mit Ammoniak bzw. primären oder sekundären Aminen ist an sich bekannt. Zwischenprodukte der Formel II, worin $W_1$ und/oder $W_2$ Cyano bedeuten, können z.B. durch partielle Hydrolyse, im Sinne einer Graf-Ritter-Reaktion, oder über Iminoniederalkylester-Salze in gegebenenfalls mono- oder disubstituierte (Mono- oder Di-)-Carbamoylverbindungen der Formel I überführt werden. Die Bedingungen bei der Hydrolyse der Cyano-Zwischenprodukte können so gewählt werden, dass die Reaktion auf der Stufe des Amids abgebrochen wird. Zu diesem Zweck ist insbesondere die Hydrolyse mit Säuren geeignet, wobei z.B. 80 %ige Schwefelsäure (unter Erwärmen), Polyphosphorsäure (bei 110-150°C), Bromwasserstoff/Eisessig (Raumtemperatur, Ameisensäure oder ohne Lösungsmittel), HCl-Gas in etherischer Lösung gefolgt von der Zugabe von Wasser oder wässriger Salzsäure, oder Borhalogenide in Frage kommen.

Mit Hilfe der Graf-Ritter-Reaktion gelingt auch die Herstellung N-substituierter Amide aus (Mono- oder Di-)Nitrilen der Formel II. Hierzu setzt man die (Mono- oder Di-)Nitrile in Gegenwart einer starken Säure, vornehmlich 85-90 %iger Schwefelsäure, oder auch Polyphosphorsäure, Ameisensäure, Bortrifluorid oder anderen Lewis-Säuren, nicht jedoch Aluminiumchlorid, mit Verbindungen um, die in dem sauren Medium Carbeniumionen bilden können, also z.B. mit Olefinen, wie Propylen, oder Alkoholen, wie Ethanol.

Die (Mono- oder Di-)Imino-niederalkylester (als Säureadditionssalze) erhält man z.B. durch säurekatalysierte Anlagerung von Alkoholen an die (Mono- oder Di-)Nitrile der Formel II. Diese Anlagerung kann gleichfalls durch Basen, z.B. Alkoholate wie Natriummethoxid, katalysiert werden, wobei man die (Mono- oder Di-)Imino-niederalkylester in freier Form erhält.

Aus den (Mono- oder Di-)Imino-niederalkylestern erhält man die (Mono- oder Di-)Amide im Sinne einer Pinner-Spaltung durch thermischen Zerfall der Iminoester-Salze bei Temperaturen oberhalb von etwa 80°C.

Andererseits können die (Mono- oder Di-)Imino-niederalkylester z.B. auch aus (Mono- oder Di-)Carbamoylverbindungen der Formel II durch Umsetzung mit Triniederalkyloxonium-tetrafluoroborat, insbesondere $^{\oplus}O(C_2H_5)_3\ BF_4^{\ominus}$ ("Meerweinsalz"), hergestellt werden.

Die (Mono- oder Di-)Imino-niederalkylthiolester werden z.B. durch S-Alkylierung der entsprechenden (Mono- oder Di-)Thiocarbamoylverbindungen (s.u.) hergestellt; zur Alkylierung können z.B. Niederalkylhalogenide oder -tosylate oder wiederum das oben erwähnte "Meerweinsalz" verwendet werden.

Die Gruppen $W_1$ und/oder $W_2$ in einer Verbindung der Formel II können bei der Herstellung von (Mono-

oder Di-)Thiocarbamoylverbindungen der Formel I (Y ≙ S und/oder Z ≙ S) z.B. bedeuten: Carbamoyl, Niederalkyl- oder Diniederalkylcarbamoyl (in diesen Fällen entspricht die Verbindung der Formel II einer Verbindung der Formel I), Cyano oder Halogencarbonyl. Die Bildung von gegebenenfalls mono- oder disubstituierten (Mono- oder Di-)Thiocarbamoylverbindungen der Formel I durch Reaktion entsprechender Zwischenprodukte der Formel II, worin $W_1$ und/oder $W_2$ z.B. wie oben angegeben definiert ist, mit Schwefel-einführenden Agentien ist an sich bekannt [vgl. z.B. Chem. Reviews 61, 45-86 (1961)]. Zwischenprodukte der Formel II, worin $W_1$ und/oder $W_2$ Carbamoyl, Niederalkyl- oder Diniederalkylcarbamoyl bedeuten, können z.B. durch Umsetzung mit Phosphorpentasulfid ($P_4S_{10}$) oder Aluminiumtrisulfid ($Al_2S_3$) oder insbesondere dem Lawesson-Reagens [2,4-Bis-(4-methoxyphenyl)-2,4-dithioxo- 1,3,2,4-dithiadiphosphetan] in gegebenenfalls mono- oder disubstituierte (Mono- oder Di-)Thiocarbamoylverbindungen der Formel I überführt werden.

Zwischenprodukte der Formel II, worin $W_1$ und/oder $W_2$ Cyano bedeuten, lassen sich z.B. durch Reaktion mit Ammoniak und Schwefelwasserstoff in Ethanol [s. z.B. Il Farmaco 41, 346-354 (1986)] oder mit Schwefelwasserstoff in Pyridin und Triethylamin [s. Beispiel 3], und solche worin $W_1$ und/oder $W_2$ Halogencarbonyl bedeuten, z.B. durch Umsetzung mit Phosphorpentachlorid, Schwefelwasserstoff und Ammoniak, in (Mono- oder Di-)-Thiocarbamoylverbindungen der Formel I überführen.

Verbindungen der Formel II, worin $W_1$ und $W_2$ Carboxy bedeuten, werden z.B. hergestellt, indem man eine Verbindung der Formel III

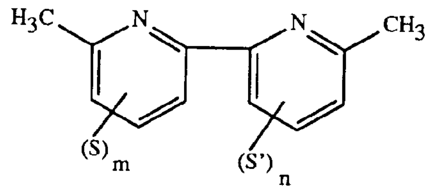

(III)

oxidiert, z.B. mit $KMnO_4$ oder $K_2Cr_2O_7$. Bedingung dabei ist, dass die Substituenten S und S′ nicht oxidationsempfindlich sein dürfen - also z.B. nicht Niederalkyl bedeuten können - oder durch Schutzgruppen gegen Oxidation geschützt sein müssen.

Weiterhin lassen sich aus Verbindungen der Formel III auch z.B. Verbindungen der Formel II, worin $W_1$ und $W_2$ Cyano bedeuten, herstellen, indem man erstere z.B. analog Chem. Pharm. Bull. 25, 1821 (1977) [≙ C.A. 88, 121089 m (1978)] mit Ethylnitrit in Gegenwart von Alkalimetallamiden in flüssigem Ammoniak zu den entsprechenden Dialdoximen nitrosiert und diese zusammen mit $POCl_3$ erhitzt.

Verbindungen der Formel II, worin $W_1$ und $W_2$ Cyano bedeuten, werden bevorzugt dadurch hergestellt, dass man eine Verbindung der Formel VI

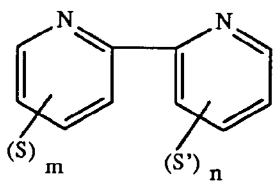

(VI)

zu einem Di-N-oxid oxidiert - z.B. mit meta-Chlorperbenzoesäure oder $H_2O_2$ [s. z.B. Bull.Chem.Soc. Japan 52, 1408 (1979)] -, dieses dimethyliert - z.B. mit Dimethylsulfat - und das erhaltene N,N′-Dimethoxyderivat mit z.B. Natriumcyanid umsetzt. Anstatt das erwähnte Di-N-oxid zu dimethylieren und mit z.B. NaCN umzusetzen, kann man es z.B. auch direkt mit Trimethylsilylcyanid (vgl. Synthesis 1984, 681 ) oder Diethylcyanophosphonat [vgl. Il Farmaco 41, 346-354 (1986)] reagieren lassen.

Verbindungen der Formel II, worin $W_1$ und $W_2$ Cyano bedeuten, werden ferner z.B. dadurch hergestellt, dass man eine Verbindung der Formel IV

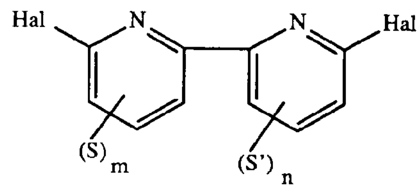

(IV)

worin Hal Halogen bedeutet, mit Cyanierungsmitteln, z.B. Natriumcyanid oder Kaliumcyanid - gegebenenfalls unter Katalyse durch $Pd[P(C_6H_5)_3]_4$ oder Phasentransferkatalyse, z.B. mit 18-Krone-6-Ether, - oder mit Kupfer(I)cyanid, insbesondere in Pyridin oder Dimethylformamid [vgl. Chem. Rev. 87, 779 (1987)], umsetzt.

Andererseits können die Halogengruppen in einer Verbindung der Formel IV auch z.B. durch Umsetzung mit Trimethylamin in Trimethylammoniumhalogenid-Reste umgewandelt werden. Letztere lassen sich dann z.B. durch Behandlung mit Natriumcyanid im wässrigen Medium in Cyanogruppen überführen, wobei wiederum Verbindungen der Formel II, worin $W_1$ und $W_2$ Cyano bedeuten, erhalten werden.

Weiterhin lassen sich aus Verbindungen der Formel IV auch z.B. Verbindungen der Formel II, worin $W_1$ und $W_2$ Carboxy bedeuten, herstellen, indem man erstere dimetalliert und dann z.B. mit $CO_2$ umsetzt. Die Metallierung kann z.B. mit Lithiierungsmitteln, z.B. n-Butyllithium, durchgeführt werden und führt dann zu Di-(Li)-Zwischenprodukten. Wird mit Lithiierungsmitteln und Kupfer(I)salzen metalliert, so erhält man Dilithiumcuprate, z.B. Di-(CuLi)-Verbindungen, als Zwischenstufen. Des weiteren kann man z.B. mit Magnesium metallieren und erhält dann Dimagnesiumhalogenide, Di-(MgHal)-Verbindungen, als Zwischenprodukte.

Verbindungen der Formel II, worin $W_1$ und $W_2$ Cyano bedeuten, können z.B. auch hergestellt werden, indem man eine Verbindung der Formel V

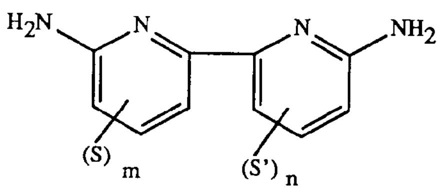

(V)

zweifach diazotiert, z.B. mit Natriumnitrit, und dann mit z.B. Kupfer(I)cyanid umsetzt (Sandmeyer-Reaktion).

Verbindungen der Formel II, worin $W_1$ und $W_2$ Cyano bedeuten, bzw. Verbindungen der Formel III, IV und VI werden z.B. hergestellt, indem man eine Verbindung der Formel VIIa

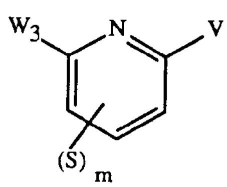

(VIIa)

mit einer Verbindung der Formel VIIb

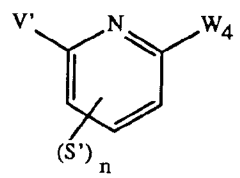

(VIIb)

umsetzt. In den Verbindungen der Formel VIIa und VIIb bedeuten $W_3$ und $W_4$ jeweils Cyano bzw. Methyl, Halogen oder Wasserstoff und V und V′ stehen für funktionelle Gruppen, die zur Verknüpfung von (Hetero-)-Arylen zu Bi-(hetero-)arylen geeignet sind. Geeignete Gruppen V und V′ sind z.B. (a) Magnesiumhalogenid -MgHal und Hal (Hal ≙ Halogen), (b) Lithium -Li und Hal oder (c) Lithiumcuprate, z.B. mit der Gruppe -CuLi, und Hal.

Ferner ist (d) die Kupplung von zwei (gleichen oder verschiedenen) Lithiumderivaten (V ≙ V′ ≙ Li) in Ge-

genwart von z.B. $CuCl_2/O_2$ oder $[CuI \cdot P(n\text{-}C_4H_9)_3]_4/O_2$ [vgl. J. Organomet. Chem. 56, 53 (1973)] zu erwähnen.

Eine weitere Möglichkeit zur Verknüpfung besteht (e) in der Kupplung einer Verbindung der Formel VIIa, worin V ≙ Li, mit einer (N-heterocyclischen) Verbindung der Formel VIIb, worin V′ ≙ H, vgl. Chem. Ber. 113, 2739 (1980).

Verbindungen der Formel V können z.B. durch Reduktion der entsprechenden Dinitroverbindungen, z.B. durch Hydrierung oder mit $Sn(II)Cl_2$, hergestellt werden. Weiterhin lassen sich Verbindungen der Formel V z.B. auch aus den entsprechenden Verbindungen der Formel IV herstellen, indem letztere mit Alkalimetallamid, z.B. Natrium- oder Kaliumamid, umgesetzt werden.

Verfahrensgemäss erhältliche freie Verbindungen der Formel I mit salzbildenden Eigenschaften können in an sich bekannter Weise in ihre Salze übergeführt werden, Verbindungen mit basischen Eigenschaften durch Behandeln mit Säuren oder geeigneten Derivaten davon, Verbindungen mit sauren Eigenschaften durch Behandeln mit Basen oder geeigneten Derivaten davon.

Infolge der engen Beziehung zwischen den Verbindungen der Formel I in freier Form und in Form von Salzen sind vor- und nachstehend unter den freien Verbindungen bzw. ihren Salzen sinn- und zweckmässig gegebenenfalls auch die entsprechenden Salze bzw. freien Verbindungen zu verstehen.

Die Verbindungen, einschliesslich ihrer Salze, können auch in Form von Hydraten erhalten werden, oder ihre Kristalle können z.B. das zur Kristallisation verwendete Lösungsmittel einschliessen.

Erfindungsgemäss erhältliche Gemische von Isomeren können in an sich bekannter Weise in die einzelnen Isomeren aufgetrennt werden, Racemate z.B. durch Bilden von Salzen mit optisch reinen salzbildenden Reagentien und Auftrennen des so erhältlichen Diastereomerengemisches, z.B. mittels fraktionierter Kristallisation.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, z. B. in Alkoholen, wie Methanol oder Ethanol, Nitrilen, wie Acetonitril, Halogenkohlenwassertoffen, wie Methylenchlorid, Wasser oder Gemischen dieser Lösungsmittel, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. bei Raumtemperatur oder im Temperaturbereich von etwa -70°C bis etwa 190°C, vorzugsweise von etwa -20°C bis etwa 150°C, z.B. beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Stickstoffatmosphäre.

Im Verfahren der vorliegenden Erfindung werden vorzugsweise solche Ausgangsstoffe eingesetzt, die zu den eingangs als besonders wertvoll beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. eines Salzes davon, verwendet wird.

Die vorliegende Erfindung betrifft ebenfalls pharmazeutische Präparate, die als Wirkstoff eine der pharmakologisch wirksamen Verbindungen der Formel I enthalten. Besonders bevorzugt sind Präparate zur enteralen, insbesondere oralen, sowie zur parenteralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Die pharmazeutischen Präparate enthalten von etwa 5 % bis etwa 95 % des Wirkstoffs, wobei einzeldosierte Applikationsformen vorzugsweise von etwa 20 % bis etwa 90 % und nicht-einzeldosierte Applikationsformen vorzugsweise etwa 5 % bis etwa 20 % Wirkstoff aufweisen. Dosiseinheits- formen, wie Dragées, Tabletten oder Kapseln, enthalten von etwa 0,05 g bis etwa 1,0 g des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, durch Zugabe von zusätzlichen Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zu parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Die Erfindung betrifft ebenfalls ein Verfahren zur Behandlung der oben genannten Krankheitszustände. Die Verbindungen der vorliegenden Erfindung können prophylaktisch oder therapeutisch verabreicht werden, beispielsweise zur Behandlung von Tumoren, die auf eine Hemmung der S-Adenosylmethionindecarboxylase ansprechen oder zur Behandlung von Protozoainfektionen bei Säugetieren, z. B. Menschen, die wegen einer derartigen Erkrankung der genannten Behandlung bedürfen, wobei man die Verbindungen der vorliegenden Erfindung vorzugsweise in Form von pharmazeutischen Präparaten verwendet.

Dabei wird bei einem Körpergewicht von etwa 70 kg eine tägliche Dosis von etwa 0,1 g bis etwa 10 g, vorzugsweise von etwa 0,5 g bis etwa 5 g einer Verbindung der vorliegenden Erfindung verabreicht.

Die nachfolgenden Beispiele illustrieren die vorliegende Erfindung; Temperaturen werden in Grad Celsius angegeben.

Beispiel 1: 6,6′-Diamidino-4,4′-dimethyl-2,2′-bipyridin-dihydrochlorid

0,023 g (0,001 g-Atom) Natrium werden unter Stickstoff in 15 ml abs. Methanol gelöst. Zu dieser Lösung werden 2,34 g (0,01 Mol) 6,6′-Dicyano-4,4′-dimethyl-2,2′-bipyridin gegeben, und das resultierende Gemisch wird 2 Tage bei Raumtemperatur gerührt. Anschliessend werden 1,34 g (0,025 Mol) Ammoniumchlorid, 10 ml abs. Methanol und 20 ml einer gesättigten ethanolischen Ammoniaklösung zugegeben, und man rührt 1 h bei 70°. Nach Abkühlen wird das Reaktionsgemisch zur Trockne eingedampft und der Rückstand aus verdünnter Salzsäure kristallisiert. Man erhält so die Titelverbindung, Smp. >240°; MS (FAB): $(M+H)^+$ = 269; 1H-NMR ($D_2O$): δ = 8,39 (d,2H); 7,88 (d,2H); 2,46 (s,6H).

Das Ausgangsmaterial wird wie folgt, entweder gemäss a1), a2) oder a3), hergestellt:

(a) 6,6'-Dicyano-4,4'-dimethyl-2,2'-bipyridin

a1) 14,25 ml (0,15 Mol) Dimethylsulfat werden bei 75° mit 12,96 g (0,06 Mol) 4,4'-Dimethyl-2,2'-bipyridin-di-N-oxid versetzt und 15 min bei 80° gehalten. Danach wird das Reaktionsgemisch abgekühlt, in 30 ml Wasser gelöst und langsam in eine eisgekühlte Lösung von 17,4 g (0,33 Mol) Natriumcyanid in 66 ml Wasser getropft. Man rührt 1 h im Eisbad nach. Das ausgefallene Material wird abgesaugt, mit Wasser gewaschen und getrocknet. Man erhält so das Ausgangsmaterial (a), Smp. 251-253°.

a2) Eine Lösung von 2,16 g (0,01 Mol) 4,4'-Dimethyl-2,2'-bipyridin-di-N-oxid in 20 ml Acetonitril und 2,23 g (0,022 Mol) Triethylamin wird tropfenweise mit 5,3 g (0,033 Mol) Diethylcyanophosphonat versetzt und 16 h am Rückfluss gekocht. Das Reaktionsgemisch wird eingedampft und mit Wasser versetzt. Das unlösliche Material wird abgesaugt, mit Wasser gewaschen, getrocknet und aus Ethanol umkristallisiert. Man erhält so das Ausgangsmaterial (a), Smp. 251-253°.

a3) Eine Lösung von 2, 16 g (0,01 Mol) 4,4'-Dimethyl-2,2'-bipyridin-di-N-oxid in 10 ml Acetonitril und 5,6 ml (0,04 Mol) Triethylamin wird mit 10 ml (0,08 Mol) Trimethylsilylcyanid versetzt und 24 h am Rückfluss gekocht. Nach dem Abkühlen wird das Reaktionsgemisch mit 100 ml Methylenchlorid verdünnt, mit verdünnter Natriumhydrogencarbonatlösung und Wasser gewaschen, über Natriumsulfat getrocknet, filtriert und eingedampft. Der Rückstand wird aus Methanol kristallisiert und entspricht dem Ausgangsmaterial (a), Smp. 251-253°, IR ($CH_2Cl_2$): 2240 $cm^{-1}$ (C=N); $^1$H-NMR ($CDCl_3$): δ = 8,52 (d,2H); 7,58 (d,2H); 2,53 (s,6H).

Beispiel 2: 6,6'-Diamidino-5,5'-dimethyl-2,2'-bipyridin-dihydrochlorid

Eine Lösung von 3,02 g (0,01 Mol) 5,5'-Dimethyl-6,6'-dithiocarbamoyl-2,2'-bipyridin (s. Beispiel 3) in 50 ml Methylenchlorid wird unter Stickstoff mit 4,3 g (0,021 Mol) Triethyloxonium-tetrafluoroborat versetzt. Nach 2 h gibt man zu der Reaktionslösung 0,98 g (0,007 Mol) Kaliumcarbonat und 0,9 ml Wasser, rührt kwz nach, filtriert und wäscht das Filtrat mit Eiswasser. Die organische Phase wird über Natriumsulfat getrocknet und eingedampft. Der rohe Bis-thioiminoether, 1,8 g (0,005 Mol), wird in 20 ml abs. Ethanol gelöst, mit 0,64 g (0,012 Mol) Ammoniumchlorid versetzt und 12 h unter Rückfluss gekocht. Nach dem Abkühlen wird filtriert, das Filtrat mit wenig ethanolischer Salzsäure versetzt und dann eingedampft. Der Rückstand wird durch Chromatographie an Amberlite® XAD 1180 (Wasser als Eluiermittel) gereinigt, wobei man die Titelverbindung erhält.

Beispiel 3: 5,5'-Dimethyl-6,6'-dithiocarbamoyl-2,2'-bipyridin

In eine Lösung von 2,34 g (0,01 Mol) 6,6'-Dicyano-5,5'-dimethyl-2,2'-bipyridin in 30 ml Pyridin und 3,0 ml (0,02 Mol) Triethylamin leitet man während 7 h und bei 40° einen trockenen Schwefelwasserstoffstrom ein. Das Reaktionsgemisch wird weitere 15 h bei 40° gerührt, abgekühlt und in Wasser gegossen. Man extrahiert mit Methylenchlorid, trocknet die organische Lösung über Natriumsulfat und dampft zur Trockne ein, wobei man die Titelverbindung erhält.

Das Ausgangsmaterial wird wie folgt hergestellt:

(a) 6,6'-Dicyano-5,5'-dimethyl-2,2'-bipyridin

14,25 ml (0,15 Mol) Dimethylsulfat werden bei 75° mit 12,96 g (0,06 Mol) 5,5'-Dimethyl-2,2'-bipyridin-di-N-oxid versetzt und 15 min bei 80° gehalten. Danach wird das Reaktionsgemisch abgekühlt, in 30 ml Wasser gelöst und langsam in eine eisgekühlte Lösung von 17,4 g (0,33 Mol) Natriumcyanid in 66 ml Wasser getropft. Man rührt 1 h im Eisbad nach. Das ausgefallene Material wird abgesaugt, mit Wasser gewaschen und getrocknet. Man erhält so die Titelverbindung.

Beispiel 4: 6,6'-Dicarbamoyl-4,4'-dimethyl-2,2'-bipyridin

0,47 g (0,002 Mol) 6,6'-Dicyano-4,4'-dimethyl-2,2'-bipyridin werden in 8 ml Ethanol und 8 ml 1N Natronlauge suspendiert und 2 h am Rückfluss gekocht. Nach dem Abkühlen wird das Reaktionsgemisch eingedampft und der Rückstand in 10 ml Wasser aufgenommen. Das unlösliche Material wird abgesaugt, mit wenig Wasser gewaschen, getrocknet und aus verdünntem Ethanol umkristallisiert. Man erhält so die Titelverbindung.

Beispiel 5: 6,6'-Bis-N-hydroxyamidino-4,4'-dimethyl-2,2'-bipyridin

Ein Gemisch von 0,47 g (0,002 Mol) 6,6'-Dicyano-4,4'-dimethyl-2,2'-bipyridin, 0,6 g (0,0056 Mol) Natriumcarbonat und 0,7 g (0,01 Mol) Hydroxylamin-hydrochlorid in 20 ml Wasser und 10 ml Ethanol wird 2 h am Rückfluss

gekocht. Nach dem Abkühlen wird das ausgefallene Material abgesaugt und aus Wasser umkristallisiert. Man erhält so die Titelverbindung, Smp. 272-276°.

Beispiel 6:

Analog zu den Beispielen 1-3 werden die folgenden Verbindungen hergestellt:

a) 6,6′-Diamidino-4,4′-dichlor-2,2′-bipyridin
b) 6,6′-Diamidino-4,4′-dimethoxy-2,2′-bipyridin
c) 6,6′-Diamidino-3,3′-dimethyl-2,2′-bipyridin
d) 6,6′-Dithiocarbamoyl-4,4′-dimethyl-2,2′-bipyridin
e) 6,6′-Bis-N-methylamidino-4,4′-dimethyl-2,2′-bipyridin
f) 6,6′-Bis-N,N-dimethylamidino-4,4′-dimethyl-2,2′-bipyridin
g) 6,6′-Bis-1-piperidinoiminomethyl-4,4′-dimethyl-2,2′-bipyridin
h) 6,6′-Bis-1-pyrrolidinoiminomethyl-4,4′-dimethyl-2,2′-bipyridin
i) 6,6′-Bis-N-cyclopentylamidino-4,4′-dimethyl-2,2′-bipyridin.

Beispiel 7:

Kapseln enthaltend 0,25 g Wirkstoff, z.B. eine der Verbindungen der Beispiele 1-6i, können wie folgt hergestellt werden:

Zusammensetzung (für 5000 Kapseln)

| | |
|---|---|
| Wirkstoff | 1250 g |
| Talk | 180 g |
| Weizenstärke | 120 g |
| Magnesiumstearat | 80 g |
| Laktose | 20 g |

Die pulverförmigen Substanzen werden durch ein Sieb mit einer Maschenweite von 0,6 mm getrieben und gemischt. Portionen von je 0,33 g des Gemisches werden mittels einer Kapselfüllmaschine in Gelatine-Kapseln abgefüllt.

## Patentansprüche

**1.** Verbindungen der Formel I

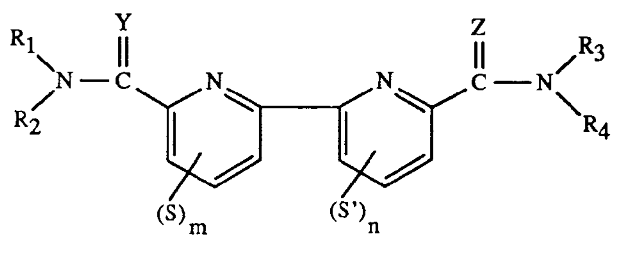

(I)

worin Y für $NR_5$, O oder S steht, Z für $NR_6$, O oder S steht, die Reste $R_2$, $R_4$, $R_5$, und $R_5$ unabhängig voneinander Wasserstoff oder Niederalkyl bedeuten und die Reste $R_1$ und $R_3$ unabhängig voneinander für Wasserstoff, Niederalkyl, Cycloalkyl, Arylniederalkyl, Aryl, freies oder funktionell abgewandeltes Carboxy, Hydroxy, verethertes oder verestertes Hydroxy oder unsubstituiertes oder mono- oder disubstituiertes Amino stehen; worin die Reste $R_1$ und $R_2$ zusammen auch Niederalkylen bedeuten können, worin die Reste $R_3$ und $R_4$ zusammen auch für Niederalkylen stehen können, worin die Reste $R_2$ und $R_5$ zusammen auch Niederalkylen bedeuten können, worin die Reste $R_4$ und $R_6$ zusammen auch für Niederalkylen stehen können; und worin S und S′ unabhängig voneinander einen von Wasserstoff verschiedenen Substituenten bedeuten; m für 0, 1, 2 oder 3 steht; n für 0, 1, 2 oder 3 steht, wobei jedoch die Summe aus m und n mindestens 1 ist; Tautomere davon, und Salze davon.

2. Verbindungen der Formel I gemäss Anspruch 1, worin Y für NH, O oder S steht, Z für NH, O oder S steht, die Reste $R_2$ und $R_4$ unabhängig voneinander Wasserstoff oder Niederalkyl bedeuten und die Reste $R_1$ und $R_3$ unabhängig voneinander für Wasserstoff, Niederalkyl, $C_3$-$C_8$-Cycloalkyl, Phenylniederalkyl, Phenyl, Carboxy, Hydroxy oder Amino stehen; worin die Reste $R_1$ und $R_2$ zusammen auch $C_2$-$C_7$-Alkylen bedeuten können, worin die Reste $R_3$ und $R_4$ zusammen auch für $C_2$-$C_7$-Alkylen stehen können; und worin S und S′ unabhängig voneinander $C_1$-$C_{20}$-Alkyl, $C_3$-$C_8$-Cycloalkyl, Phenylniederalkyl, $C_3$-$C_8$-Cycloalkylniederalkyl, Phenyl, $C_1$-$C_{20}$-Alkoxy, Phenylniederalkoxy, Phenyloxy, Halogen oder Niederalkylthio bedeuten; m für 0 oder 1 steht; und n für 0 oder 1 steht; wobei jedoch die Summe aus m und n mindestens 1 ist; wobei in den obigen Definitionen Phenylgruppen unsubstituiert oder durch Niederalkyl, Hydroxy, Niederalkoxy, Halogen und/oder Trifluormethyl substituiert sind; Tautomere davon, und Salze davon.

3. Verbindungen der Formel I gemäss Anspruch 1, worin Y für NH, O oder S steht, Z für NH, O oder S steht, die Reste $R_2$ und $R_4$ unabhängig voneinander Wasserstoff oder Niederalkyl bedeuten und die Reste $R_1$ und $R_3$ unabhängig voneinander für Wasserstoff, Niederalkyl, $C_3$-$C_8$-Cycloalkyl, Phenylniederalkyl, Phenyl, Carboxy, Hydroxy oder Amino stehen; worin die Reste $R_1$ und $R_2$ zusammen auch $C_2$-$C_7$-Alkylen bedeuten können, worin die Reste $R_3$ und $R_4$ zusammen auch für $C_2$-$C_7$-Alkylen stehen können; und worin S und S′ unabhängig voneinander Niederalkyl, Phenylniederalkyl, Niederalkoxy, Halogen oder Niederalkylthio bedeuten; m für 0 oder 1 steht; und n für 0 oder 1 steht; wobei jedoch die Summe aus m und n mindestens 1 ist; Tautomere davon, und Salze davon.

4. Verbindungen der Formel I gemäss Anspruch 1, worin Y für NH, O oder S steht, Z für NH, O oder S steht, die Reste $R_2$ und $R_4$ Wasserstoff oder Niederalkyl bedeuten, die Reste $R_1$ und $R_3$ für Wasserstoff, Niederalkyl, $C_5$-$C_6$-Cycloalkyl oder Hydroxy stehen, S und S′ Niederalkyl, Niederalkoxy oder Halogen bedeuten, und m und n für 1 stehen; Tautomere davon, und pharmazeutisch verwendbare Salze davon.

5. Verbindungen der Formel I gemäss Anspruch 1, worin Y für NH steht, Z für NH steht, die Reste $R_2$ und $R_4$ Wasserstoff bedeuten, die Reste $R_1$ und $R_3$ für Wasserstoff, Niederalkyl, $C_5$-$C_6$-Cycloalkyl oder Hydroxy stehen, S und S′ in 4- bzw. 4′-Stellung angeknüpft sind und Niederalkyl, Niederalkoxy oder Halogen bedeuten, und m und n für 1 stehen, Tautomere davon, und pharmazeutisch verwendbare Salze davon.

6. Verbindungen der Formel I gemäss Anspruch 5, worin $R_1$ und $R_3$ Wasserstoff bedeuten, Tautomere davon, und pharmazeutisch verwendbare Salze davon.

7. 6,6′-Diamidino-4,4′-dimethyl-2,2′-bipyridin gemäss Anspruch 1 oder ein pharmazeutisch verwendbares Salz davon.

8. 6,6′-Diamidino-5,5′-dimethyl-2,2′-bipyridin gemäss Anspruch 1 oder ein pharmazeutisch verwendbares Salz davon.

9. 6,6′-Diamidino-4,4′-dimethoxy-2,2′-bipyridin gemäss Anspruch 1 oder ein pharmazeutisch verwendbares Salz davon.

10. 6,6′-Diamidino-3,3′-dimethyl-2,2′-bipyridin gemäss Anspruch 1 oder ein pharmazeutisch verwendbares Salz davon.

11. 6,6′-Bis-N-hydroxyamidino-4,4′-dimethyl-2,2′-bipyridin gemäss Anspruch 1 oder ein pharmazeutisch verwendbares Salz davon.

12. Pharmazeutische Präparate enthaltend eine Verbindung der Formel I, ein Tautomeres davon, oder ein Salz davon gemäss einem der Ansprüche 1 bis 11 und mindestens ein pharmazeutisch verwendbares Trägermaterial.

13. Eine Verbindung der Formel I, ein Tautomeres davon, oder ein Salz davon gemäss einem der Ansprüche 1 bis 11 zur Anwendung in einem Verfahren zur therapeutischen Behandlung des tierischen oder menschlichen Körpers.

14. Verwendung von einer Verbindung der Formel I, einem Tautomeren davon, oder einem Salz davon gemäss einem der Ansprüche 1 bis 11 zur Herstellung pharmazeutischer Präparate zur Anwendung für die

Behandlung von Protozoainfektionen.

15. Verfahren zur Herstellung einer Verbindung der Formel I eines Tautomeren davon, oder eines Salzes davon gemäss Anspruch 1, dadurch gekennzeichnet, dass man in einer Verbindung der Formel II

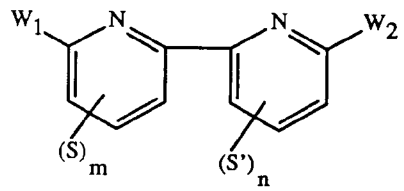

(II)

worin $W_1$ und $W_2$ jeweils einen in die Gruppe $-C(=Y)NR_1R_2$ bzw. $-C(=Z)NR_3R_4$ überführbaren Rest bedeuten, die Reste $W_1$ und $W_2$ in die Gruppe $-C(=Y)NR_1R_2$ bzw. $-C(=Z)NR_3R_4$ überführt; wobei die Symbole Y, Z, $R_1$-$R_4$, S, S′, m und n die unter Formel I angegebene Bedeutung haben; und gewünschtenfalls eine erhaltene Verbindung der Formel I in eine andere Verbindung der Formel I umwandelt, und/oder ein erhaltenes Salz in die freie Verbindung oder in ein anderes Salz umwandelt, und/oder eine erhaltene freie Verbindung der Formel I mit salzbildenden Eigenschaften in ein Salz umwandelt.

**Patentansprüche für folgende Vertragsstaaten : ES, GR**

1. Verfahren zur Herstellung einer Verbindung der Formel I

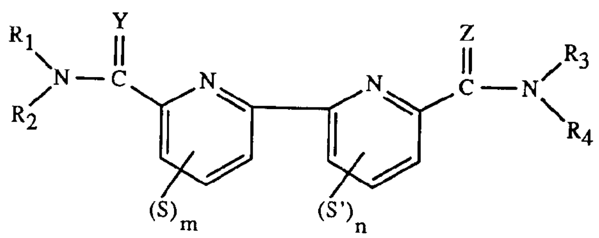

(I)

worin Y für $NR_5$, O oder S steht, Z für $NR_6$, O oder S steht, die Reste $R_2$, $R_4$, $R_5$, und $R_5$ unabhängig voneinander Wasserstoff oder Niederalkyl bedeuten und die Reste $R_1$ und $R_3$ unabhängig voneinander für Wasserstoff, Niederalkyl, Cycloalkyl, Arylniederalkyl, Aryl, freies oder funktionell abgewandeltes Carboxy, Hydroxy, verethertes oder verestertes Hydroxy oder unsubstituiertes oder mono- oder disubstituiertes Amino stehen; worin die Reste $R_1$ und $R_2$ zusammen auch Niederalkylen bedeuten können, worin die Reste $R_3$ und $R_4$ zusammen auch für Niederalkylen stehen können, worin die Reste $R_2$ und $R_5$ zusammen auch Niederalkylen bedeuten können, worin die Reste $R_4$ und $R_6$ zusammen auch für Niederalkylen stehen können; und worin S und S′ unabhängig voneinander einen von Wasserstoff verschiedenen Substituenten bedeuten; m für 0, 1, 2 oder 3 steht; n für 0, 1, 2 oder 3 steht, wobei jedoch die Summe aus m und n mindestens 1 ist; von Tautomeren davon, und von Salzen davon, dadurch gekennzeichnet, dass man in einer Verbindung der Formel II

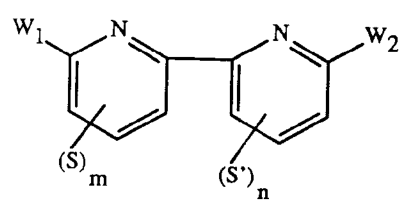

(II)

worin $W_1$ und $W_2$ jeweils einen in die Gruppe $-C(=Y)NR_1R_2$ bzw. $-C(=Z)NR_3R_4$ überführbaren Rest bedeuten, die Reste $W_1$ und $W_2$ in die Gruppe $-C(=Y)NR_1R_2$ bzw. $-C(=Z)NR_3R_4$ überführt; wobei die Symbole Y, Z, $R_1$-$R_4$, S, S′, m und n die unter Formel I angegebene Bedeutung haben; und gewünschtenfalls eine erhaltene Verbindung der Formel I in eine andere Verbindung der Formel I umwandelt, und/oder ein erhaltenes Salz in die freie Verbindung oder in ein anderes Salz umwandelt, und/oder eine erhaltene freie Verbindung der Formel I mit salzbildenden Eigenschaften in ein Salz umwandelt.

**2.** Verfahren gemäss Anspruch 1 zur Herstellung von Verbindungen der in Anspruch 1 gezeigten Formel I, worin Y für NH, O oder S steht, Z für NH, O oder S steht, die Reste $R_2$ und $R_4$ unabhängig voneinander Wasserstoff oder Niederalkyl bedeuten und die Reste $R_1$ und $R_3$ unabhängig voneinander für Wasserstoff, Niederalkyl, $C_3$-$C_8$-Cycloalkyl, Phenylniederalkyl, Phenyl, Carboxy, Hydroxy oder Amino stehen; worin die Reste $R_1$ und $R_2$ zusammen auch $C_2$-$C_7$-Alkylen bedeuten können, worin die Reste $R_3$ und $R_4$ zusammen auch für $C_2$-$C_7$-Alkylen stehen können; und worin S und S′ unabhängig voneinander $C_1$-$C_{20}$-Alkyl, $C_3$-$C_8$-Cycloalkyl, Phenylniederalkyl, $C_3$-$C_8$-Cycloalkylniederalkyl, Phenyl, $C_1$-$C_{20}$-Alkoxy, Phenylniederalkoxy, Phenyloxy, Halogen oder Niederalkylthio bedeuten; m für 0 oder 1 steht; und n für 0 oder 1 steht; wobei jedoch die Summe aus m und n mindestens 1 ist; wobei in den obigen Definitionen Phenylgruppen unsubstituiert oder durch Niederalkyl, Hydroxy, Niederalkoxy, Halogen und/oder Trifluormethyl substituiert sind; von Tautomeren davon, und von Salzen davon, dadurch gekennzeichnet, dass man entsprechend substituierte Ausgangsmaterialien verwendet.

**3.** Verfahren gemäss Anspruch 1 zur Herstellung von Verbindungen der in Anspruch 1 gezeigten Formel I, worin Y für NH, O oder S steht, Z für NH, O oder S steht, die Reste $R_2$ und $R_4$ unabhängig voneinander Wasserstoff oder Niederalkyl bedeuten und die Reste $R_1$ und $R_3$ unabhängig voneinander für Wasserstoff, Niederalkyl, $C_3$-$C_8$-Cycloalkyl, Phenylniederalkyl, Phenyl, Carboxy, Hydroxy oder Amino stehen; worin die Reste $R_1$ und $R_2$ zusammen auch $C_2$-$C_7$-Alkylen bedeuten können, worin die Reste $R_3$ und $R_4$ zusammen auch für $C_2$-$C_7$-Alkylen stehen können; und worin S und S′ unabhängig voneinander Niederalkyl, Phenylniederalkyl, Niederalkoxy, Halogen oder Niederalkylthio bedeuten; m für 0 oder 1 steht; und n für 0 oder 1 steht; wobei jedoch die Summe aus m und n mindestens 1 ist; von Tautomeren davon, und von Salzen davon, dadurch gekennzeichnet, dass man entsprechend substituierte Ausgangsmaterialien verwendet.

**4.** Verfahren gemäss Anspruch 1 zur Herstellung von Verbindungen der in Anspruch 1 gezeigten Formel I, worin Y für NH, O oder S steht, Z für NH, O oder S steht, die Reste $R_2$ und $R_4$ Wasserstoff oder Niederalkyl bedeuten, die Reste $R_1$ und $R_3$ für Wasserstoff, Niederalkyl, $C_5$-$C_6$-Cycloalkyl oder Hydroxy stehen, S und S′ Niederalkyl, Niederalkoxy oder Halogen bedeuten, und m und n für 1 stehen; von Tautomeren davon, und von pharmazeutisch verwendbaren Salzen davon, dadurch gekennzeichnet, dass man entsprechend substituierte Ausgangsmaterialien verwendet.

**5.** Verfahren gemäss Anspruch 1 zur Herstellung von Verbindungen der in Anspruch 1 gezeigten Formel I, worin Y für NH steht, Z für NH steht, die Reste $R_2$ und $R_4$ Wasserstoff bedeuten, die Reste $R_1$ und $R_3$ für Wasserstoff, Niederalkyl, $C_5$-$C_6$-Cycloalkyl oder Hydroxy stehen, S und S′ in 4- bzw. 4′-Stellung angeknüpft sind und Niederalkyl, Niederalkoxy oder Halogen bedeuten, und m und n für 1 stehen, von Tautomeren davon, und von pharmazeutisch verwendbaren Salzen davon, dadurch gekennzeichnet, dass man entsprechend substituierte Ausgangsmaterialien verwendet.

**6.** 6. Verfahren gemäss Anspruch 1 zur Herstellung von Verbindungen der in Anspruch 1 gezeigten Formel I, worin $R_1$ und $R_3$ Wasserstoff bedeuten, von Tautomeren davon, und von pharmazeutisch verwendbaren Salzen davon, dadurch gekennzeichnet, dass man entsprechend substituierte Ausgangsmaterialien verwendet.

**7.** Verfahren gemäss Anspruch 1 zur Herstellung von einer Verbindung der in Anspruch 1 gezeigten Formel I mit dem Namen 6,6′-Diamidino-4,4′-dimethyl-2,2′-bipyridin oder einem pharmazeutisch verwendbaren Salz davon, dadurch gekennzeichnet, dass man entsprechend substituierte Ausgangsmaterialien verwendet.

**8.** Verfahren gemäss Anspruch 1 zur Herstellung von einer Verbindung der in Anspruch 1 gezeigten Formel I mit dem Namen 6,6′-Diamidino-5,5′-dimethyl-2,2′-bipyridin oder einem pharmazeutisch verwendbaren Salz davon, dadurch gekennzeichnet, dass man entsprechend substituierte Ausgangsmaterialien verwendet.

**9.** Verfahren gemäss Anspruch 1 zur Herstellung von einer Verbindung der in Anspruch 1 gezeigten Formel I mit dem Namen 6,6′-Diamidino-4,4′-dimethoxy-2,2′-bipyridin oder einem pharmazeutisch verwendbaren Salz davon, dadurch gekennzeichnet, dass man entsprechend substituierte Ausgangsmaterialien verwendet.

**10.** Verfahren gemäss Anspruch 1 zur Herstellung von einer Verbindung der in Anspruch 1 gezeigten Formel

I mit dem Namen 6,6′-Diamidino-3,3′-dimethyl-2,2′-bipyridin oder einem pharmazeutisch verwendbaren Salz davon, dadurch gekennzeichnet, dass man entsprechend substituierte Ausgangsmaterialien verwendet.

**11.** Verfahren gemäss Anspruch 1 zur Herstellung von einer Verbindung der in Anspruch 1 gezeigten Formel I mit dem Namen 6,6′-Bis-N-hydroxyamidino-4,4′-dimethyl-2,2′-bipyridin oder einem pharmazeutisch verwendbaren Salz davon, dadurch gekennzeichnet, dass man entsprechend substituierte Ausgangsmaterialien verwendet.

**12.** Verfahren zur Herstellung eines pharmazeutischen Präparates, dadurch gekennzeichnet, dass man eine gemäss einem Verfahren eines der Ansprüche 1 bis 11 erhaltene Verbindung der Formel I, ein Tautomeres davon, oder ein Salz davon mit mindestens einem pharmazeutisch verwendbaren Trägermaterial versetzt.

**13.** Verfahren zur Herstellung eines pharmazeutischen Präparates, dadurch gekennzeichnet, dass man eine gemäss einem Verfahren eines der Ansprüche 1 bis 11 erhaltene Verbindung der Formel I, ein Tautomeres davon, oder ein Salz davon mit mindestens einem pharmazeutisch verwendbaren Trägermaterial versetzt, wobei der Wirkstoff im erhaltenen Präparat in einem Gewichtsanteil von 5 bis 95 % vorliegt.

Europäisches Patentamt

# EUROPÄISCHER RECHERCHENBERICHT

Nummer der Anmeldung

EP 92 81 0003

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | EP-A-0 350 448 (CIBA-GEIGY AG) 10. Januar 1990<br>* das ganze Dokument *<br>--- | 1-15 | C07D213/81<br>A61K31/44<br>C07D213/78<br>C07D213/83 |
| A | CHEMICAL ABSTRACTS, vol. 105, no. 9, 1. September 1986, Columbus, Ohio, US; abstract no. 72085R, I. ANTONINI ET AL.: '2,2'-Bipyridyl-6-carbothioamide derivatives as potential antitumor agents' Seite 16 ;<br>* Zusammenfassung *<br>& FARMACO, ED. SCI. Bd. 41, Nr. 5, 1986, Seiten 346 - 354;<br>--- | 1,12,13 | |
| A | CHEMICAL ABSTRACTS, vol. 105, no. 11, 15. September 1992, Columbus, Ohio, US; abstract no. 90903, G. CRISTALLI ET AL.: '2,2-Bipyridyl-6-carboxamidoximes with potential antitumor and antimicrobial properties.' Seite 30 ;<br>* Zusammenfassung *<br>& FARMACO, ED. SCI. Bd. 41, Nr. 7, 1986, Seiten 499 - 507;<br>----- | 1,12,13 | RECHERCHIERTE SACHGEBIETE (Int. Cl.5 )<br>C07D<br>A61K |

Der vorliegende Recherchenbericht wurde für alle Patentansprüche erstellt

| Recherchenort | Abschlußdatum der Recherche | Prüfer |
|---|---|---|
| DEN HAAG | 11 MAERZ 1992 | DE JONG B.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)